# FASCICULE DE BREVET EUROPEEN

(11) **EP 4 347 009 B1**
(45) Date de publication et mention de la délivrance du brevet: **03.09.2025**
(21) Numéro de dépôt: 22730932.5
(22) Date de dépôt: 03.06.2022
(51) Int. Cl.: A61N 2/02, A61N 2/00

(54) **SYSTÈME DE STIMULATION ÉLECTROMAGNÉTIQUE**
SYSTEM ZUR ELEKTROMAGNETISCHEN STIMULATION
SYSTEM FOR ELECTROMAGNETIC STIMULATION

(30) Priorité: 04.06.2021 FR 2105879
(43) Date de publication de la demande: 10.04.2024
(73) Titulaire: Deleo, 83600 FREJUS (FR)
(72) Inventeur: ABOU SALEH, Anthony, 83480 PUGET-SUR-ARGENS (FR); BERTHON, Alexandre, 83700 SAINT-RAPHAEL (FR)
(74) Mandataire: Hautier IP
(86) Numéro de dépôt international: PCT/EP2022/065205
(87) Numéro de publication internationale: WO 2022/254015

(56) Documents cités:
- WO-A2-2004/098700
- US-A1- 2011 004 261
- US-A1- 2011 105 828
- US-A1- 2014 358 197
- US-A1- 2015 071 978
- US-A1- 2015 360 045
- US-A1- 2016 220 834
- US-A1- 2020 001 103
- US-A1- 2020 046 038

## Description

### DOMAINE TECHNIQUE DE L'INVENTION

La présente invention concerne le domaine des protocoles esthétiques. Elle trouve pour application particulièrement avantageuse les stimulations électromagnétiques à haute intensité des tissus sous-cutanés, destinées à redessiner les lignes du corps de façon ciblée et harmonieuse. La présente invention concerne une solution technique permettant une utilisation optimisée des appareils de soins esthétiques.

### ETAT DE LA TECHNIQUE

Il existe de nombreuses solutions permettant de sculpter la silhouette. Notamment, parmi ces solutions, certaines sont considérées comme ne nécessitant aucun effort puisqu'elles utilisent des dispositifs de stimulation des tissus sous-cutanés à l'aide d'applicateurs.

Les appareils de « body contouring » que l'on peut traduire comme des appareils sculptant un contour de corps idéal sous forme de machines à stimulation électromagnétique, produisent des ondes électromagnétiques indolores pour le corps qui ne nécessitent pas d'anesthésie. Les ondes électromagnétiques activent les motoneurones afin de permettre la contraction des muscles. En faisant varier les paramètres d'intensité, il est possible ainsi d'adapter le protocole afin d'atteindre des résultats de musculation que l'on souhaite.

L'électrostimulation permet ainsi d'activer les cellules et de développer la masse musculaire. Pour une meilleure efficacité de ce type de protocole esthétique, l'applicateur électromagnétique doit être positionné au plus près de la surface de la peau. Ainsi, l'activation des cellules est optimale. Néanmoins, l'application d'un champ magnétique au contact direct de la peau, entraîne inévitablement une chauffe des tissus sous-cutanée, et induit chez la personne un effet de sudation. De plus, le champ électromagnétique va faire vibrer les molécules d'eau contenues dans la sueur et faire chauffer fortement la peau et cela provoque généralement des brûlures superficielles.

Pour pallier ce problème, on rencontre des solutions comme par exemple le document US10471269B1 dans lequel est divulgué un dispositif pour traiter au moins une région corporelle d'une personne par des impulsions magnétiques répétées. Dans ce type de dispositif, les courants de Foucault induits par la génération de champ magnétique, conduisent à la production de chaleur indésirable et nécessitent par conséquent un élément de refroidissement contre la peau de la personne recevant le protocole esthétique. Le document US 2020/001103 A1 divulgue un système selon le préambule de la revendication 1.

Néanmoins, ce type de solutions présente un véritable désavantage car elles sont complexes et coûteuses à mettre en œuvre.

Il existe donc un besoin d'amélioration de l'application d'un dispositif de stimulation électromagnétique sur la peau du corps humain.

Un objet de la présente invention est donc de proposer une solution qui permette de supprimer ou de limiter au moins l'un des inconvénients précités.

Les autres objets, caractéristiques et avantages de la présente invention apparaîtront à l'examen de la description suivante et des dessins d'accompagnement. Il est entendu que d'autres avantages peuvent être incorporés.

### RESUME DE L'INVENTION

Pour atteindre cet objectif, selon un mode de réalisation on prévoit un système configuré pour permettre la stimulation des tissus sous cutanée du corps d'une personne, le système comprenant une machine de stimulation électromagnétique à haute densité configurée pour émettre des ondes électromagnétiques, la machine comprenant :
- une unité centrale configurée pour être alimenté électriquement,
- un applicateur comprenant :
   - une portion distale apte à être positionnée sur une région du corps et émettre les ondes électromagnétique dans une configuration de stimulation,
   - une portion de branchement, opposée à la portion distale et configurée pour être reliée via une connexion filaire à l'unité centrale,
   - une enveloppe vestimentaire en tissu.

L'enveloppe vestimentaire en tissu est apte, dans une configuration de stimulation :
- à être portée par la personne au moins au contact de la région du corps,
- à être intercalée entre l'applicateur et la région du corps.

Le système selon l'invention est caractérisé en ce que l'enveloppe vestimentaire comprend un taux d'élasthanne supérieur à 5%, un taux de coton supérieur à 20% et couvre simultanément les muscles abdominaux et les muscles fessiers de la personne.

Sans la présente invention il faudrait prévoir comme suggéré dans l'art antérieur, un dispositif de refroidissement couplé à l'applicateur pour faire baisser la température superficielle de la région du corps stimulée.

Ainsi, le système permet d'éviter un contact direct entre l'applicateur et la peau et évite toutes brûlures potentielles. L'enveloppe vestimentaire permettant une dissipation de l'humidité.

De ce fait, le système permet d'appliquer des ondes électromagnétiques à proximité de la peau d'une personne en contrôlant avec précision l'espace entre la peau et l'applicateur.

La présente solution permet donc préférentiellement un protocole esthétique optimisé et à moindre coût. L'enveloppe vestimentaire en tissu permet d'optimiser le parcours de l'applicateur sur au moins une région du corps.

L'invention concerne un système tel que défini par les revendications jointes. Les exemples ou les modes de réalisation non couverts par la portée des revendications jointes, notamment les procédés, doivent être considérés uniquement comme des exemples permettant de comprendre l'invention.

Selon un mode de réalisation, la divulgation comprend un procédé d'application d'un protocole esthétique permettant la stimulation des tissus sous cutanée du corps d'une personne, à l'aide d'un système comprenant :
- une machine de stimulation électromagnétique à haute densité configurée pour émettre des ondes électromagnétiques,
- une enveloppe vestimentaire en tissu,
le procédé comprenant les étapes suivantes :
- positionnement de l'enveloppe vestimentaire au contact d'une région du corps de la personne,
- positionnement d'un applicateur de la machine contre l'enveloppe vestimentaire de sorte à ce que l'enveloppe vestimentaire soit intercalée entre l'applicateur et la région du corps.

Un autre aspect concerne l'utilisation d'une enveloppe vestimentaire en tissu dans le cadre d'un protocole esthétique, l'enveloppe vestimentaire étant configurée pour s'intercaler entre un applicateur d'une machine de stimulation électromagnétique à haute densité émettant des ondes électromagnétiques et une région du corps d'une personne, l'enveloppe vestimentaire étant configurée pour être portée par la personne au contact de la région du corps.

### BREVE DESCRIPTION DES FIGURES

Les buts, objets, ainsi que les caractéristiques et avantages de l'invention ressortiront mieux de la description détaillée d'un mode de réalisation de cette dernière qui est illustré par les dessins d'accompagnement suivants dans lesquels :

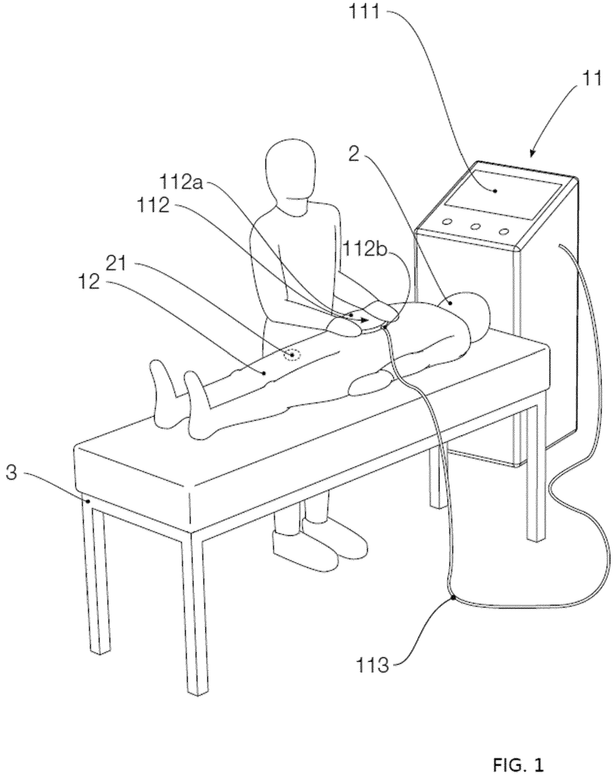

La figure 1 représente une personne utilisant le système, avec l'applicateur au niveau des abdominaux.

Les dessins sont donnés à titre d'exemples et ne sont pas limitatifs de l'invention. Ils constituent des représentations schématiques de principe destinées à faciliter la compréhension de l'invention et ne sont pas nécessairement à l'échelle des applications pratiques.

### DESCRIPTION DÉTAILLÉE

Avant d'entamer une revue détaillée de modes de réalisation de l'invention, sont énoncées ci-après des caractéristiques optionnelles qui peuvent éventuellement être utilisées en association ou alternativement.

Selon un exemple, l'enveloppe vestimentaire 12 présente un volume interne libre lorsqu'elle n'est pas portée et présente un volume interne porté lorsqu'elle est portée par la personne 2 dans la configuration de stimulation, le volume interne porté étant supérieur au volume interne libre.

Ainsi, l'enveloppe vestimentaire 12 épouse de manière optimisée au moins une région du corps 21 de la personne 2. Cela répond à la problématique consistant à positionner de manière homogène, l'enveloppe vestimentaire 12 au contact de la peau sans qu'il n'y ait d'espaces ou de plis entre l'enveloppe et la peau à proximité de la région du corps 21 stimulée.

Selon un exemple, l'enveloppe vestimentaire 12 présente une épaisseur régulière et est venue d'une unique matière de sorte à présenter une élasticité isotrope.

Cela permet un positionnement de l'applicateur à une distance constante de la peau et une efficacité régulière du protocole quel que soit le parcours de l'applicateur 112.

Selon un exemple, l'enveloppe vestimentaire 12 est en une matière qui ne comprend pas d'élément métallique. En effet, lors de l'application d'un champ électromagnétique à haute densité, les éléments ferromagnétiques seraient fortement attirés et/ou repoussés par aimantation. De plus, les métaux sont généralement connus pour leur très bonne conductivité thermique. Par conséquent, des éléments métalliques pourraient être la cause de brûlures pour la personne 2 recevant le protocole.

Selon un exemple, l'enveloppe vestimentaire 12 présente une épaisseur constante e telle que e < 3 mm, de préférence e < 1 mm, de préférence e < 0,8 mm, de préférence e = 0,5 mm. Selon l'invention, l'enveloppe vestimentaire 12 comprend un taux d'élasthanne supérieur à 5%, de préférence supérieur à 10%, de préférence égal à 15 %.

Selon l'invention, l'enveloppe vestimentaire 12 comprend un taux de coton supérieur à 20%, de préférence supérieur à 30%, de préférence égal à 40 %.

Selon un exemple, l'applicateur 112 est configuré pour émettre une force électromagnétique allant de 2 à 10 Tesla, une fréquence allant de 1 à 80 Hertz pour des périodes d'impulsion allant entre 100 et 600 µs.

Selon un exemple, l'enveloppe vestimentaire 12 comprend des mailles textiles dont l'ajourement est configuré pour s'agrandir dès lors que l'enveloppe vestimentaire 12 est portée. Cela permet une adaptation de l'enveloppe textile 12 sur les différents types de morphologies humaines.

Selon un exemple, l'enveloppe vestimentaire 12 est un vêtement qui recouvre au moins 50 % de la surface du corps de la personne 2.

Selon un exemple, l'enveloppe vestimentaire 12 est une combinaison.

Selon un exemple, l'enveloppe vestimentaire 12 est configurée pour présenter une capacité d'allongement sous l'action d'une tension, comprise entre 10 et 30 millimètres lorsque la tension exercée est comprise entre 0,30 et 3 Newtons.

Il est précisé que dans le cadre de la présente invention, le terme « tissu » correspond à un textile ou une étoffe obtenue par l'assemblage de fils entrelacés.

On entend par « tissus sous-cutané », l'ensemble des couches inférieures à la peau comprenant des graisses et/ou des cellules.

Dans la présente invention, le terme « au niveau de » s'entend comme situé à proximité de l'élément visé, ou dans une zone proche de.

Il est précisé que, dans le cadre de la présente invention, les termes « sur », « surmonte », « recouvre », « sous-jacent », en « vis-à-vis » et leurs équivalents ne signifient pas forcément « au contact de ».

Dans la description qui suit, sauf indication contraire, lorsque l'on fait référence à des qualificatifs de position absolue, tels que les termes "avant", "arrière", "haut", "bas", "gauche", "droite", etc., ou relative, tels que les termes "dessus", "dessous", "supérieur", "inférieur", etc., ou à des qualificatifs d'orientation, tels que les termes "horizontal", "vertical", "latéral", etc., il est fait référence à l'orientation des figures correspondantes, étant entendu que, dans la pratique, les dispositifs et assemblages décrits peuvent être orientés différemment.

De même lorsqu'on indique qu'un élément est situé au droit d'un autre élément, cela signifie que ces deux éléments sont situés sur une même ligne perpendiculaire au plan principal dans lequel s'étend principalement une face du substrat, c'est-à-dire sur une même ligne orientée verticalement sur les figures.

Un élément situé « à l'aplomb » ou « au droit » d'un autre élément signifie que ces deux éléments sont situés tous deux sur une même ligne perpendiculaire à un plan dans lequel s'étend principalement une surface inférieure ou supérieure.

Les termes « sensiblement », « environ », « de l'ordre de » signifient « à 10% près, de préférence à 5% près »

Lorsqu'il s'agit d'un taux de matière défini en pourcentage, il pourra être ajusté à 5% près.

### SYSTÈME DE STIMULATION ELECTROMAGNETIQUE - APPROCHE STRUCTURELLE

Selon un mode de réalisation en particulier, le système est utilisé dans le cadre de protocoles esthétiques d'une personne 2 et comprend une machine 11 et une enveloppe vestimentaire 12.

### MACHINE

De manière préférée, la machine 11 comprend une unité centrale 111 et un applicateur 112. Avantageusement, l'applicateur 112 et l'unité centrale 111 sont reliés par une connexion filaire pouvant par exemple être un câble de connexion 113. La machine 11 est configurée de sorte à émettre un champ électromagnétique au contact ou à proximité d'une enveloppe vestimentaire 12 portée par une personne. La machine 11 est ainsi configurée de sorte à permettre des contractions musculaires forcées d'une personne 2 lorsque l'applicateur 112 est situé au moins à proximité, voire au contact, de la région du corps 21.

L'action des ondes électromagnétiques permet avantageusement de tonifier les muscles et de faire diminuer la masse graisseuse de la personne 2.

La machine 11 comprend préférentiellement une alimentation électrique de puissance et/ou des condensateurs et/ou une unité de calcul et/ou une carte électronique de commande et un applicateur 112.

Selon un mode de réalisation particulier, la machine 11 est configurée de sorte à émettre des ondes électromagnétiques de préférence à haute densité, sur les muscles de la personne 2 via l'applicateur 112 qu'il y ait ou non un contact direct entre la personne 2 et l'applicateur 112.

### L'APPLICATEUR

L'applicateur 112 comprend par exemple un bobinage métallique, de préférence en cuivre. L'applicateur 112 étant configuré de sorte à pouvoir émettre un champ électromagnétique à haute densité. Le bobinage étant préférentiellement intégré au sein d'une coque protectrice qui peut être faite en matière plastique.

De manière préférée, le bobinage est en spirale et est couplé à une alimentation électrique de sorte à générer un champ électromagnétique de haute intensité et de faible fréquence. Cela permet notamment la génération d'un champ électromagnétique suffisamment puissant pour traverser au moins partiellement une région musculaire du corps 21 de manière non invasive et non ablative en interagissant avec les neurones moteurs afin de déclencher des contractions musculaires forcées et intenses.

Avantageusement, la puissance du champ électromagnétique permettant de réaliser ce type de contractions musculaires est comprise entre 0,5 et 8 Tesla et la fréquence des impulsions est comprise entre 1 et 100 Hertz.

Selon un mode de réalisation de la présente invention, la bobine comprend un fil conducteur en cuivre configuré de sorte à transporter un courant électrique variable afin de générer un champ magnétique dont l'amplitude et la direction en chaque point de l'espace dépend de la longueur et de la forme du fil, du courant et de l'emplacement du point où le champ est déterminé. Préférentiellement, l'applicateur comprend une bobine circulaire comportant une multitude de tours autour d'un axe d'enroulement, ainsi un champ électromagnétique est induit selon l'axe d'enroulement de la bobine.

Selon un mode de réalisation, l'applicateur 112 comprend au moins une face active, configurée de sorte à émettre des ondes électromagnétiques selon des lignes de champs. L'au moins une face active de l'applicateur 112 est avantageusement lisse et/ou courbe, configurée de sorte à pouvoir être positionnée au contact de la personne 2 et préférentiellement épouser les formes d'au moins une région de corps 21. Avantageusement, le bobinage est placé à l'intérieur d'une cavité de l'applicateur. De manière préférée, le bobinage est disposé à l'intérieur d'une coque de l'applicateur 112, la coque pouvant être en plastique souple ou solide.

### UNITE CENTRALE

Avantageusement, l'unité centrale 111 de la machine 11 est connectée à une source électrique de puissance. L'unité centrale 111 est configurée de sorte à se connecter à l'applicateur 112 par l'intermédiaire d'une connexion filaire 113. Ainsi, l'unité centrale 111 agit comme un générateur qui génère un courant électrique à l'applicateur 112 via l'élément de connexion filaire 113. Une fois dans l'applicateur 112, le courant électrique traverse la bobine et génère un champ magnétique induit.

### CONNECTEUR

L'élément de connexion filaire 113 peut être un câble électrique intégré dans une gaine protectrice.

### ENVELOPPE VESTIMENTAIRE : TYPOLOGIE

Selon un mode de réalisation de la présente invention, l'enveloppe vestimentaire 12 est un vêtement pouvant-être porté par la personne 2 et est apte à recouvrir au moins partiellement une région de son corps 21. Il pourra s'agir d'un vêtement configuré pour être porté et recouvrir par exemple, plus de 30% de la surface du corps, de préférence plus de 50 % de la surface du corps.

Selon l'invention, l'enveloppe vestimentaire 12 couvre simultanément les muscles abdominaux et les muscles fessiers de la personne 2. Avantageusement l'enveloppe vestimentaire 12 est une combinaison. L'enveloppe vestimentaire 12 revêt de préférence, au moins une portion de la région du corps 21. De préférence, l'enveloppe vestimentaire 12 ceint la région du corps 21 de sorte à former une section fermée.

### MATIERE ELASTIQUE ET CONTACT CONTINU

Selon un mode de réalisation préféré de la présente invention, l'enveloppe vestimentaire 12 est en tissu en ce qu'elle comprend au moins des mailles tissées. Préférentiellement, l'enveloppe vestimentaire 12 comprend au moins une surface souple et résistante constituée par un assemblage régulier de fils entrelacés, tissés ou à mailles. Selon un mode de réalisation, l'enveloppe vestimentaire 12 est venue d'une matière comprenant au moins un matériau élastique de sorte à pouvoir assurer un contact constant et homogène de l'enveloppe vestimentaire 12 avec la peau d'au moins une région du corps 21 lorsqu'elle est portée par la personne 2. Avantageusement, le matériau élastique comprend une fibre élastique comme par exemple de l'élasthanne avec un taux supérieur à 5%, de préférence supérieur à 10%, de préférence égal à 15 %.

Selon un mode de réalisation particulier, le tissu comprend des fibres utilisées en chaîne et formant une trame régulière. De manière préférée, l'enveloppe vestimentaire 12 est configurée pour s'étendre élastiquement lorsqu'elle est portée, de préférence la maille est configurée pour s'étendre d'au moins 10%. Avantageusement, l'enveloppe vestimentaire 12 comprend des mailles configurées pour s'allonger lorsqu'une tension est exercée sur l'enveloppe vestimentaire 12 et revenir dans son état initial lorsque la tension est arrêtée.

Selon un mode de réalisation, l'enveloppe vestimentaire 12 présente un volume interne libre lorsqu'elle n'est pas portée et présente un volume interne porté lorsqu'elle est portée par la personne 20 dans la configuration de stimulation, le volume interne porté étant supérieur au volume interne libre. Avantageusement, le volume interne portée est au moins 10% plus grand que le volume interne libre, de préférence au moins 20 % plus grand, de préférence au moins 30% plus grand.

### MATIERE ABSORBANTE

Selon un mode de réalisation, l'enveloppe vestimentaire 12 est faite d'une matière comprenant au moins un matériau absorbant de sorte à pouvoir absorber l'eau contenue dans la sueur de la personne 2. Avantageusement, le matériau absorbant comprend du coton. Le matériau absorbant peut également comprendre un parmi les matériaux suivants : du chanvre, de la microfibre, de la viscose de bambou, ou du Tencel ^{®}. De manière préférée, l'enveloppe vestimentaire 12 comprend un taux de coton supérieur à 20%, de préférence supérieur à 30%, de préférence égal à 40 %.

### MATIERE FINE EPAISSEUR CONSTANTE

De manière préférée, l'enveloppe vestimentaire 12 est un vêtement venu d'une seule et même matière et qui présente une épaisseur constante. Avantageusement, l'épaisseur est inférieure à 5 mm, de préférence inférieure à 3 mm, de préférence inférieure à 1 mm, de préférence inférieure ou égale à 0,5 mm.

### MATIERE RESISTANTE ET LAVABLE

Selon un mode de réalisation particulier, l'enveloppe vestimentaire 12 comprend un matériau structurant pouvant être avantageusement des fibres polyamides de sorte à permettre une structure rigide, résistante et lavable. Avantageusement, le taux de fibre de polyamide est supérieur à 20%, de préférence égal à 35%. En effet, l'utilisation d'une matière résistante permet préférentiellement, une réutilisation de ladite enveloppe vestimentaire 2.

### ENVELOPPE VESTIMENTAIRE SANS ELEMENTS METALLIQUES

De manière préférée, l'enveloppe vestimentaire 12 comprend une matière ne comportant pas d'éléments métalliques ou ferromagnétiques. En effet, les éléments métalliques étant fortement conducteur de chaleur, ils sont susceptibles de chauffer très fortement et d'entraîner des brûlures chez la personne 2.

### SYSTÈME - APPROCHE FONCTIONNELLE

Selon un mode de réalisation préféré de la présente description, le protocole esthétique consiste à approcher l'applicateur 112 de l'enveloppe vestimentaire 12 recouvrant et/ou au contact d'au moins une région du corps 21. Les stimulations électromagnétiques émises par la machine 11 stimulent les tissus sous-cutanés et les muscles de la personne 2.

Selon l'invention, l'enveloppe vestimentaire 12 comprend un matériau absorbant configuré de sorte à absorber la sudation de la personne 2 afin d'empêcher la formation de poches d'eau sur la surface de la région du corps 21, susceptibles de chauffer sous l'action du champ électromagnétique.

Selon l'invention, l'enveloppe vestimentaire 12 comprend un matériau élastique de sorte à ce que l'enveloppe vestimentaire 12 se déforme lorsqu'elle est portée afin d'épouser les courbes de la région du corps 21.

Le système de la présente invention a pour objet une application esthétique et exclut tout traitement thérapeutique.

### REFERENCES NUMERIQUES

1. système
11. machine
111. unité centrale
112. applicateur
112a. portion distale
112b. portion de branchement
113. câble
2. personne
21. région du corps
3. support

## Revendications

1. Système configuré pour permettre la stimulation des tissus sous cutanée du corps (21) d'une personne (2), le système comprenant une machine (11) de stimulation électromagnétique configurée pour émettre des ondes électromagnétiques, la machine (11) comprenant :
- une unité centrale (111) configurée pour être alimentée électriquement,
- un applicateur (112) comprenant :
- une portion distale (112a) apte à être positionnée sur une région du corps (21) et émettre les ondes électromagnétiques dans une configuration de stimulation,
- une portion de branchement (112b), opposée à la portion distale (112a) et configurée pour être reliée via une connexion filaire (113) à l'unité centrale (111),
le système comportant une enveloppe vestimentaire (12) en tissu, apte, dans une configuration de stimulation :
- à être portée par la personne (2) au moins au contact de la région du corps (21),
- à être intercalée entre l'applicateur (112) et la région du corps (21),
le système étant **caractérisé en ce que** l'enveloppe vestimentaire (12) comprend un taux d'élasthanne supérieur à 5%, un taux de coton supérieur à 20% et couvre simultanément les muscles abdominaux et les muscles fessiers de la personne (2).

2. Système selon la revendication précédente dans lequel l'enveloppe vestimentaire (12) présente un volume interne libre lorsqu'elle n'est pas portée et présente un volume interne porté lorsqu'elle est portée par la personne (2) dans la configuration de stimulation, le volume interne porté étant supérieur au volume interne libre.

3. Système selon l'une quelconque des revendications précédentes dans lequel l'enveloppe vestimentaire (12) présente une épaisseur régulière et est venue d'une unique matière de sorte à présenter une élasticité isotrope.

4. Système selon l'une quelconque des revendications précédentes dans lequel l'enveloppe vestimentaire (12) est faite d'une matière qui ne comprend pas d'élément métallique.

5. Système selon l'une quelconque des revendications précédentes dans lequel l'enveloppe vestimentaire (12) présente une épaisseur constante e telle que e < 3 mm, de préférence e < 1 mm, de préférence e = 0,5 mm.

6. Système selon l'une quelconque des revendications précédentes dans lequel l'applicateur (112) est configuré pour émettre une force électromagnétique allant de 2 à 10 Tesla, une fréquence allant de 1 à 80 Hertz pour des périodes d'impulsion allant entre 100 et 600 µs.

7. Système selon l'une quelconque des revendications précédentes dans lequel l'enveloppe vestimentaire (12) comprend des mailles textiles dont l'ajourement est configuré pour s'agrandir dès lors que l'enveloppe vestimentaire (12) est portée.

8. Système selon l'une quelconque des revendications précédentes dans lequel l'enveloppe vestimentaire (12) est un vêtement qui recouvre au moins 50 % de la surface totale du corps de la personne (2).

9. Système selon l'une quelconque des revendications précédentes dans lequel l'enveloppe vestimentaire (12) est une combinaison.

10. Système selon l'une quelconque des revendications précédentes dans lequel l'enveloppe vestimentaire (12) est configurée pour présenter une capacité d'allongement sous l'action d'une tension, comprise entre 10 et 30 millimètres lorsque la tension exercée est comprise entre 0,30 et 3 Newtons.

## Patentansprüche

1. System, das so eingerichtet ist, dass es die Stimulation des Unterhautgewebes des Körpers (21) einer Person (2) ermöglicht, wobei das System eine elektromagnetische Stimulationsmaschine (11) umfasst, die so eingerichtet ist, dass sie elektromagnetische Wellen aussendet, wobei die Maschine (11) Folgendes umfasst:
- eine Zentraleinheit (111), die so eingerichtet ist, dass sie elektrisch versorgt wird,
- einen Applikator (112), umfassend:
- einen distalen Abschnitt (112a), der geeignet ist, auf einem Körperbereich (21) positioniert zu werden und die elektromagnetischen Wellen in einer Stimulationskonfiguration auszusenden,
- einen Anschlussabschnitt (112b), der dem distalen Abschnitt (112a) gegenübersteht und so eingerichtet ist, dass er über eine drahtgebundene Verbindung (113) mit der Zentraleinheit (111) verbunden ist,
wobei das System eine geeignete Kleidungshülle (12) aus Stoff in einer Stimulationskonfiguration aufweist:
- von der Person (2) mindestens in Kontakt mit dem Körperbereich (21) getragen werden,
- zwischen dem Applikator (112) und dem Körperbereich (21) eingelegt werden,
wobei das System **dadurch gekennzeichnet ist, dass** die Kleidungshülle (12) einen Elastangehalt von mehr als 5 %, einen Baumwollgehalt von mehr als 20 % umfasst und gleichzeitig die Bauchmuskeln und die Gesäßmuskeln der Person (2) bedeckt.

2. System nach dem vorhergehenden Anspruch, wobei die Kleidungshülle (12) ein freies Innenvolumen besitzt, wenn sie nicht getragen wird, und ein getragenes Innenvolumen besitzt, wenn sie von der Person (2) in der Stimulationskonfiguration getragen wird, wobei das getragene Innenvolumen größer ist als das freie Innenvolumen.

3. System nach einem der vorhergehenden Ansprüche, wobei die Kleidungshülle (12) eine gleichmäßige Dicke besitzt und aus einem einzigen Material besteht, sodass sie eine isotrope Elastizität besitzt.

4. System nach einem der vorhergehenden Ansprüche, wobei die Kleidungshülle (12) aus einem Material besteht, das kein Metallelement umfasst.

5. System nach einem der vorhergehenden Ansprüche, wobei die Kleidungshülle (12) eine konstante Dicke e wie e < 3 mm, vorzugsweise e < 1 mm, vorzugsweise e = 0,5 mm, besitzt.

6. System nach einem der vorhergehenden Ansprüche, wobei der Applikator (112) so eingerichtet ist, dass er eine elektromagnetische Kraft im Bereich von 2 bis 10 Tesla, eine Frequenz im Bereich von 1 bis 80 Hertz für Impulsperioden im Bereich von 100 bis 600 µs abgibt.

7. System nach einem der vorhergehenden Ansprüche, wobei die Kleidungshülle (12) Textilgewirke umfasst, deren Lochung so eingerichtet ist, dass sie sich vergrößert, sobald die Kleidungshülle (12) getragen wird.

8. System nach einem der vorhergehenden Ansprüche, wobei die Kleidungshülle (12) ein Kleidungsstück ist, das mindestens 50 % der gesamten Körperfläche der Person (2) bedeckt.

9. System nach einem der vorhergehenden Ansprüche, wobei die Kleidungshülle (12) eine Kombination ist.

10. System nach einem der vorhergehenden Ansprüche, wobei die Kleidungshülle (12) so eingerichtet ist, dass sie unter Einwirkung einer Spannung eine Dehnungsfähigkeit zwischen 10 und 30 Millimetern besitzt, wenn die ausgeübte Spannung zwischen 0,30 und 3 Newton liegt.

## Claims

1. System configured to enable stimulation of the subcutaneous tissues of the body (21) of a person (2), the system comprising an electromagnetic stimulation machine (11) configured to emit electromagnetic waves, the machine (11) comprising:
- a central unit (111) configured to be powered electrically,
- an applicator (112) comprising:
- a distal portion (112a) able to be positioned on a region of the body (21) and to emit the electromagnetic waves in a stimulation configuration,
- a connection portion (112b), opposite the distal portion (112a) and configured to be connected via a wired connection (113) to the central unit (111),
the system including a fabric garment envelope (12), suitable, in a stimulation configuration:
- to be worn by the person (2) at least in contact with the region of the body (21),
- to be interposed between the applicator (112) and the region of the body (21),
the system being **characterised in that** the garment envelope (12) has an elastane content of above 5%, a cotton content of above 20% and simultaneously covers the abdominal muscles and the gluteal muscles of the person (2).

2. System according to the preceding claim, wherein the garment envelope (12) has a free internal volume when it is not being worn and has a worn internal volume when it is worn by the person (2) in the stimulation configuration, the worn internal volume being greater than the free internal volume.

3. System according to any one of the preceding claims, wherein the garment envelope (12) has a uniform thickness and is made from a single material so as to have isotropic elasticity.

4. System according to any one of the preceding claims, wherein the garment envelope (12) is made from a material that does not include any metallic elements.

5. System according to any one of the preceding claims, wherein the garment envelope (12) has a constant thickness e, such that e < 3 mm, preferably e < 1 mm, preferably e = 0.5 mm.

6. System according to any one of the preceding claims, wherein the applicator (112) is configured to emit an electromagnetic force ranging from 2 to 10 Tesla, a frequency ranging from 1 to 80 Hertz for pulse periods ranging from 100 to 600 µs.

7. System according to any one of the preceding claims, wherein the garment envelope (12) comprises textile meshes whose openings are configured to enlarge when the garment envelope (12) is worn.

8. System according to any one of the preceding claims, wherein the garment envelope (12) is a garment that covers at least 50% of the total surface area of the person's body (2).

9. System according to any one of the preceding claims, wherein the garment envelope (12) is a suit.

10. System according to any one of the preceding claims, wherein the garment envelope (12) is configured to have an elongation capacity under a tension of between 10 and 30 millimetres when the tension exerted is between 0.30 and 3 Newtons.
